# EUROPEAN PATENT APPLICATION

(11) **EP 1 013 770 A1**
(43) Date of publication of application: **28.06.2000**
(21) Application number: 98124578.0
(22) Date of filing: 23.12.1998
(51) Int. Cl.: C12N 15/79, C12N 15/85

(54) **Non-viral transfection vector**

(71) Applicant: Université Louis Pasteur de Strasbourg, F-67000 Strasbourg (FR)
(72) Inventor: Behr, Jean Paul, Dr., 67100 Strasbourg (FR); Belguise-Valladier, Pascale, 67550 Vendenheim (FR); Zanta, Maria-Antonietta, 67000 Strasbourg (FR)
(74) Representative: Laudien, Dieter, Dr.

(57) **Abstract**

A non-viral transfection vector which is to be delivered into the nucleus of a cell comprises a DNA molecule that is equipped with 1 to 15 NLS conjugates. Each conjugate comprises a nuclear localization signal (NLS) covalently linked to an oligonucleotide. The NLS conjugate may be covalently linked to one or both termini of a linear DNA molecule, associated with a plasmid DNA molecule by forming a triple helix, or inserted in a plasmid DNA molecule by strand invasion. The transfection vector is useful for gene therapy applications.

## Description

The present invention relates to the field of non-viral gene delivery.

Translocation of exogeneous DNA through the nuclear membrane has been shown to be a major problem in gene delivery technologies.
The nuclear membrane of eukaryotic cells is freely permeable to solutes of size up to ca. 9 nm (e.g. 40-60 kDa proteins). Transport of larger molecules through nuclear pores is signal-mediated, involves shuttle molecules and requires energy. The basic peptide derived from the SV40 large T antigen (PKKKRKV) is a nuclear localization signal (NLS) that mediates binding of the karyophilic protein to importin-α (Adam and Gerace, 1991.
Complex formation triggers binding to importin-β and the ternary complex is then carried through the nuclear pore with the help of the GTPase Ran. Macromolecules and particles up to 25 nm have been shown to enter the nucleus in this way, although the translocation mechanism is still not fully understood (Panté and Aebi, 1995; Nigg, 1997; Ohno et al., 1998; Melchior and Gerace, 1998).

Eukaryotic DNA viruses which replicate in the nucleus seem to be capable of diverting the cell's nuclear import machinery to their own benefit (Greber et al., 1997; Greber, 1998). Recombinant viruses derived therefrom are being used to carry therapeutic genes into humans. However, the host's immune response is currently a limitation to the clinical development of viral gene therapy. Nonviral alternatives using plasmid DNA and cationic carrier molecules suffer from a different drawback, the low efficacy of gene delivery. The main barrier to transgene expression *in vitro*, is the nuclear membrane (Zabner et al., 1995; Labat-Moleur et al., 1996; Hagstrom et al. 1997). Since breakdown of this membrane during cell division helps nuclear localization, this obstacle is probably still more a problem *in vivo*, where cells can be considered as resting with respect to the lifetime of DNA.

Several attempts to improve entry of plasmid DNA into the nucleus have been suggested. These include use of electrostatic binding of DNA to cationic NLS-containing proteins (Kaneda et al., 1989; Fritz et al., 1996) or peptides (Collas et al., 1996) or lipids (Remy et al., 1995)), as well as sequence-specific binding of DNA to karyophilic proteins (Fominaya, J. & Wels, W. 1996; Langle-Rouault et al., 1998). Yet such "piggyback" nuclear transport relies on the unpredictable stability of the complexes within the cytoplasm. Recent work by the group of Jon Wolff (Sebestyén et al., 1998) with digitonin-permeabilized cells demonstrated nuclear accumulation of fluorescently-labelled DNA that was randomly tagged with hundreds of NLS peptides covalently attached to the DNA; nuclei of intact cells did not take up the modified DNA.

It was an object of the present invention to overcome the problems of the previous methods of nuclear transport of DNA and to provide an improved gene delivery system.

The present invention is directed to a non-viral transfection vector comprising a DNA molecule which is to be delivered into the nucleus of a cell, characterized that said DNA molecule is equipped with 1-15 conjugates comprising a nuclear localization signal covalently linked to an oligonucleotide, each of which NLS-conjugate has been
i) covalently linked to one or both termini of a linear DNA molecule, or
ii) associated with a plasmid DNA molecule by forming a triple helix, or
iii) inserted in a plasmid DNA molecule by strand invasion.
In the following, the nuclear localization signal is termed "NLS".
NLSs are peptides which are known to be required for nuclear transport of karyophilic proteins. They are part of the primary structure of most nuclear proteins and contain one or two clusters of basic amino acids. Typically, they are six to eighteen amino acids in length.

A wide range of NLSs from various organisms has been characterized, examples of NLSs that can be used in the present invention are given in WO 95/31557, which is incorporated herein by reference in its entirety.

The NLS must fulfil the requirements to direct the DNA or the protein encoded by the DNA, respectively, to the nucleus.

Depending on the organism whose cells are to be transfected, e.g. an animal or a plant, the NLS is selected from a karyophilic protein expressed by that organism or from a virus infectious for that organism.

A preferred NLS used in the present invention is the NLS of the SV40 virus large T antigen.

The NLS peptide can be synthesized according to standard methods of peptide chemistry. In general,the peptide is identical to the naturally occuring NLS. However, if desired, e.g. to improve the nuclear transporting function of the peptide, the peptide may be modified, e.g. by replacing one or more of the basic amino acids by other basic amino acids. Another NLS modification, which has been used in the experiments of the present invention, is the extension of the NLS beyond its actual nuclear localization domain by the N-terminal amino acids of a karyophilic protein, preferably the authentic protein that the NLS originates from. The peptide may carry an amino acid such as cysteine, lysine, tyrosine, threonine, serine that allows convenient linkage of an oligonucleotide.

Generally, the length of the NLS is six to sixty, preferably six to fourty, even more preferably six to thirty amino acids.

In order to assess whether a selected NLS is suitable for the cell to be transfected, i.e. that it directs the protein of interest into the nucleus, assays are conducted which are known in the art, e.g. those described by Garcia-Bustos et al., 1991; Sandler et al., 1989; Citovsky et al., 1992; Sebestyén et al., 1998). These assays are based on the detection of the protein in the nucleus, e.g. by histochemical methods. A useful assay involves the use of a reporter gene, e.g the luciferase gene; the delivery of the luciferase is detected by standard luciferase detection methods as described in the Examples of the present invention.
Preferably, the NLS contains a reactive thiol group from a cysteine residue and is conjugated to the oligonucleotide by conventional methods, e.g. by amino-modifying the oligonucleotide and linking the two compounds with bifunctional cross linkers like SMCC (4-(N-maleimidomethyl)-cyclohexane-1-carboxlic acid N-hydroxysuccinimide ester). Alternative coupling methods that can be used in the present invention, e.g. activation of the 5'-phosphate groups of the oligonucleotide with imidazole or tetrazole in the presence of a carbodiimide compound and subsequent dislacement of that group by the terminal amino group of the NLS, are disclosed in WO 88/05077, which is incorporated herein by reference in its entirety.

Preferably, the DNA molecule is equipped with 1 to 5 NLS conjugates.

In the embodiment i) of the present invention, the DNA molecule is linear. The oligonucleotide part of the conjugate has a sequence that allows to form a hairpin and has, preferably at its 5'-end, a cohesive extension that is ligated, by standard cloning methods, to a complementary cohesive generated at the end of the DNA molecule. The linear DNA molecule carries one or two NLSs covalently linked to the hairpin at the 5' and/or at 3' terminus of the DNA molecule.

This hairpin-forming oligonucleotide is preferably 15 to 60 nucleotides in length. The NLS is conjugated to the oligonucleotide by coupling the peptide to a amino-modified nucleotide in the loop of the hairpin.

Preferably, in the embodiment i) of the invention, only one terminus of the linear DNA molecule carries an oligonucleotide hairpin which has an NLS coupled to it, the other terminus of the DNA molecule is capped with an identical or different oligonucleotide hairpin that does not carry an NLS. In the experiments of the present invention, is has been surprisingly shown that a single NLS is sufficient to carry DNA to the nucleus.

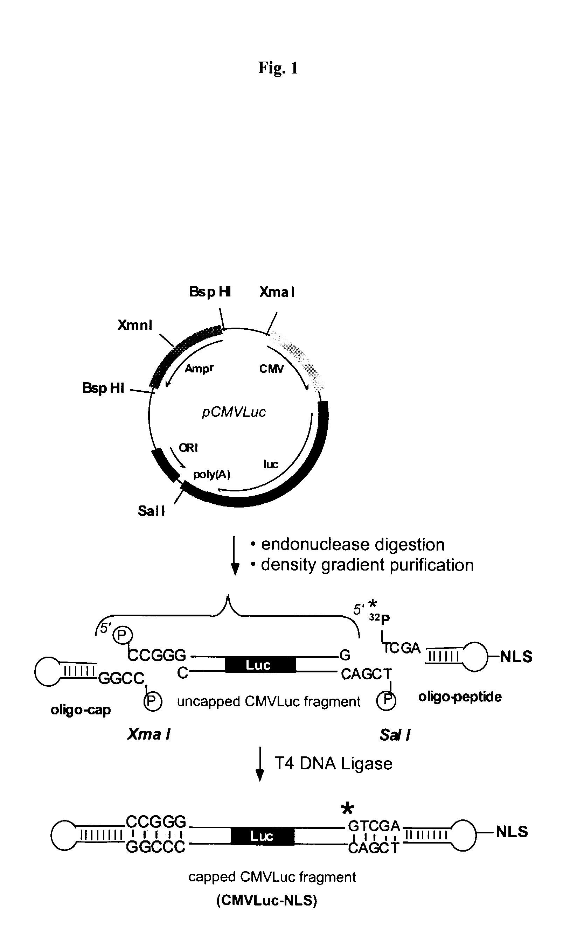

Figure 1 depicts an example for the strategy for the preparation of a dsDNA fragment coupled to an NLS peptide. A functional luciferase gene of 3380 bp was cut out of pCMVLuc with *Xma*I and *Sal*I. Further digestion with *Xmn*I and *Bsp*HI cut the unwanted restriction fragment into small fragments (970, 875, 768 and 240 bp) which were removed by sucrose gradient centrifugation. The capped CMVLuc-NLS DNA was obtained by ligation of the ³²P radioactive (*) oligo-peptide and oligo-cap hairpins to the restriction fragment

The following considerations have lead to the design of the nuclear transport system according to embodiment i) of the invention:

When fully counterion-condensed, a single plasmid molecule collapses into a sphere of ca. 25 nm (Blessing et al., 1998)) which is already at the size-exclusion limit for signal-mediated nuclear import (Ohno et al., 1998; Gorlich and Mattaj,1996). Plasmid condensation with cationic lipids or polymers generally leads to even larger, multimolecular aggregates which reach the cytoplasm after binding to cell-surface anionic proteoglycans (Labat-Moleur et al., 1996; Mislick and Baldeschwieler, 1996) and eventual escape from the formed vacuoles (Zabner et al., 1995; Plank et al., 1994; Boussif et al., 1995). Since the particles are too large to cross an intact nuclear membrane, transfection of resting cells can reasonably only be accounted for by uncomplexed DNA that has been released by exchange with phosphatidylserine (Xu and Szoka, 1996) or heparan sulfate (Labat-Moleur et al., 1996) present in the vacuolar membrane. Any molecular information (e.g. NLS) borne by the cationic vector will thus be lost prior to reaching the nuclear membrane, hence its ineffectiveness.

In sharp contrast to a condensed DNA molecule, a free hydrated DNA double helix (3 nm) is thin enough to enter the nuclear pore by diffusion, i.e. with no energy nor signal requirement. Restricted intracellular motion of DNA (the cytoplasm is equivalent to 13% dextran (Luby-Phelps, 1994)) and a rather low nuclear pore coverage (<10% of the membrane surface (Maul and Deaven, 1977)) however give this event a low probability to occur: the nuclear membrane has been recognized as a major barrier to gene delivery (Zabner et al., 1995; Labat-Moleur et al., 1996). Quantitative cytoplasmic microinjection of DNA (Pollard et al., 1998; Mirzayans et al.,1992; Dowty et al., 1995) indeed led to less than 0.1% of the DNA being expressed. The probability for DNA to find and subsequently enter a nuclear pore can be increased by a bound karyophilic signal peptide able to dock DNA to the nuclear pore filaments and help an initial part of the molecule to cross the membrane. The DNA-karyophilic signal link needs however be stable, therefore the peptide was chemically linked to the oligonucleotide.

The linear DNA molecule approach i), which is exemplified in the experiments of the invention, takes advantage of a chemically controlled pathway to irreversibly link a NLS-peptide to one end of a gene. In the experiments of the inventions, in contrast to the state of the art, the level of transgene expression following transfection was chosen as a test of success.

The major achievement of the linear DNA/NLS approach is that, for the first time using a nonviral technology, the amount of DNA required to effectively transfect cells in vitro has shifted from the microgram to the nanogram range. Although the improvement (1-3 orders of magnitude) varies with cell type (showing that the nuclear membrane is not the only possible barrier), even the modest enhancements observed for primary cell cultures look interesting (1000%) when not presented on a logarithmic scale. To show that this result was a consequence of the involvement of the nuclear import machinery, a Lys ⇒ Thr mutation was used which indeed abolished the beneficial effect brought about by the DNA-bound NLS sequence.

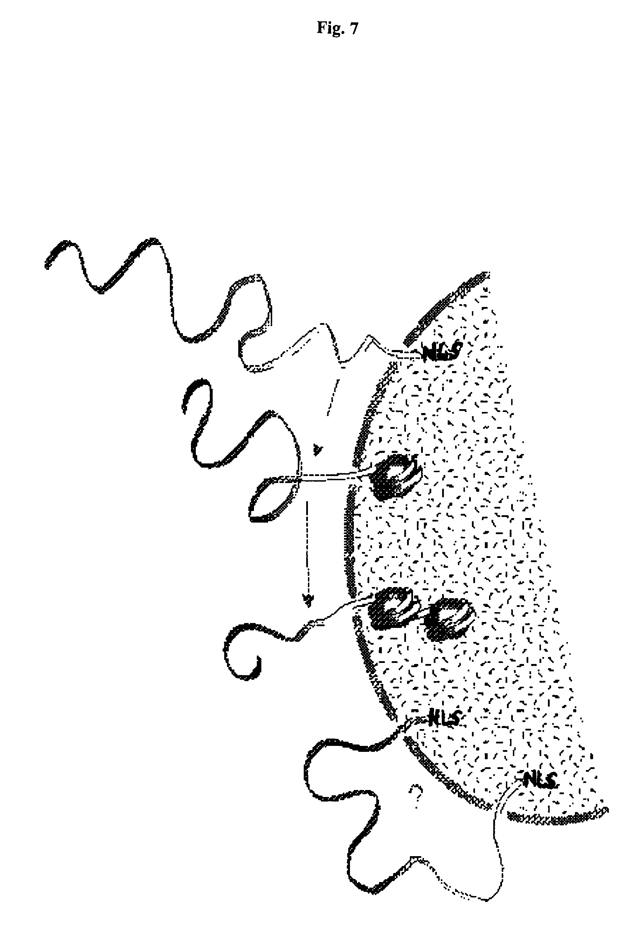

Taking the raised hypothesis further, the last unanswered question deals with threading of the rest of the plasmid molecule (Figure 7). Random-walk diffusion of a micrometer-long molecule would be inefficient and slow. Fortunately, naked DNA will not remain free in the nucleus : histones (and eventually basic nuclear matrix proteins) indeed quickly assemble transfected DNA into chromatin-like structures (Cereghini and Yaniv, 1984; Jeong and Stein, 1994), thus providing a mechanism for pulling and condensing the filamentous molecule into the nucleus. Following this line of thinking, too many NLS signals distributed along the DNA may actually inhibit nuclear entry if the nucleic acid is longer than the distance separating adjacent pores (Fig. 7). A straightforward calculation using the pore density in HeLa cells (Maul and Deaven, 1977) shows this to become probable above 1 kbp.

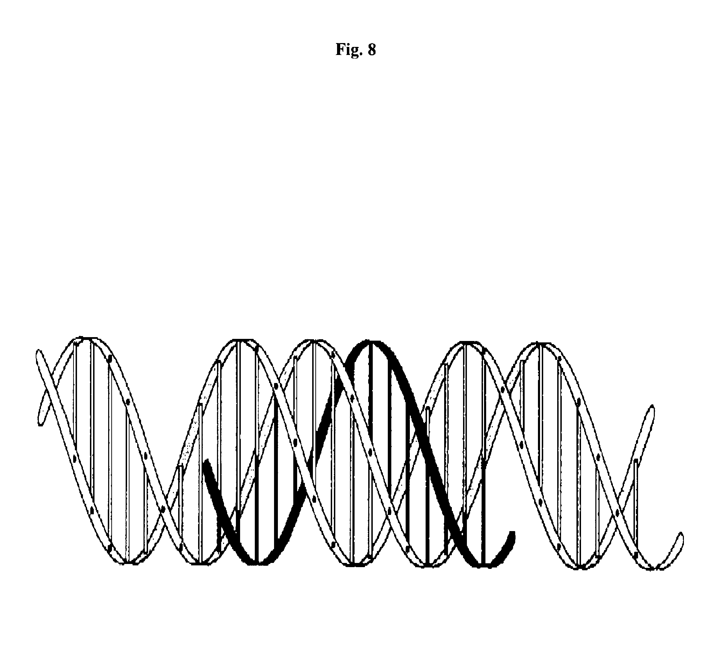

In embodiment ii) of the invention, the DNA molecule is a plasmid encoding any protein of interest, and the NLS peptide is covalently linked to a oligonucleotide that recognizes a homopurine/homopyrimidine sequence within the plasmid in a sequence specific manner (Le Doan et al., 1987; Moser and Dervan, 1987). The interaction of the oligonucleotide with the duplex DNA takes place in the major groove leading to a local triple helix structure (Fig. 8). The use of a triple-helix structure to obtain strong association between a duplex molecule and an oligonucleotide is an attractive method to generate the NLS-bearing plasmid molecule.

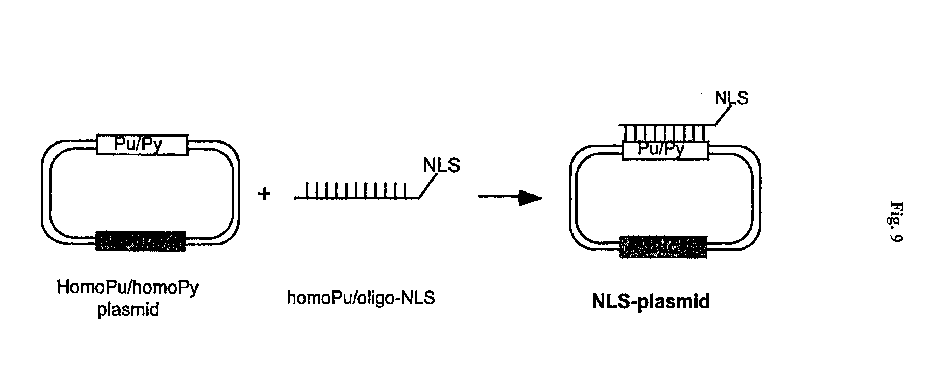

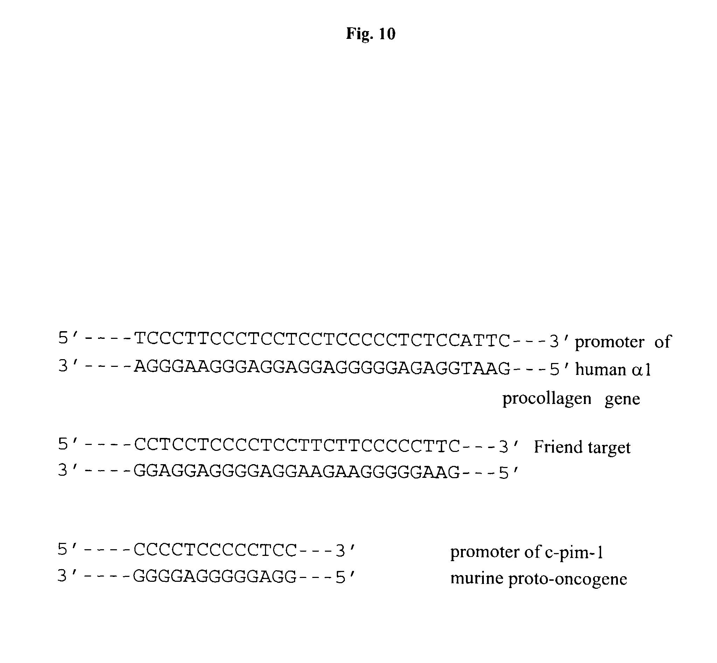

Two families of DNA triple helices have been characterized that differ in their third-strand sequence composition and relative orientation. In the pyrimidine family (Py*Pu-Py), a homopyrimidine strand (Py) is bound (*) parallel to the purine strand of target duplex through Hoogsteen base pairing. In the purine family (Pu*Pu-Py), a homopurine strand (Pu) is bound in an anti-parallel orientation to the purine strand of target duplex through reverse Hoogsteen base pairing. In the purine family, it has been shown that G-rich homopurine oligonucleotides are able to form triple helices with high stability compatible with physiological conditions (Debin et al., 1997; Nakanishi et al., 1998). This type of sequence, which is preferred in this embodiment of the invention, leads to a stable non-covalent association between the NLS-oligonucleotide and the plasmid (Fig. 9).
The selected homopurine-homopyrimidine oligonucleotide sequence can be introduced in the plasmid by conventional cloning methods, e.g. as described in Sambrook et al., 1989, at a specific position outside of the promoter region, outside of the gene coding for the protein of interest, and of the polyadenylation signal. Preferably, the oligonucleotide, which in this embodiment of the invention is a triple-helix-forming oligonucleotide (TFO), is between 8 and 40 nucleotides long. Some examples of G-rich sequences, that are useful for this embodiment of the invention, e.g. a nearly perfect 45mer polypurine tract localized in the gag gene of Friend murine leukemia virus (F-MuLV), are presented in Fig. 10 (Debin et al., 1997; Nakanishi et al., 1998; Svinarchuk et al., 1994; Rando et al., 1994).

It has been reported for several G-rich homoPu sequences that triple helix association can persist inside cells for 1 to 3 days (Debin et al., 1997; Svinarchuk et al., 1994). Therefore, for appropriate homoPu-homo-Py sequences, the NLS-oligo/plasmid triplexes are expected to be stable enough to remain undissociated inside the cell until the triplex is carried to the nucleus. Furthermore, by introducing several identical homoPu-homo-Py sequences in the plasmid, the influence of both number and distance between NLS's on nuclear import can be assessed in order to optimize the design of the construct.

The NLS peptide is covalently linked to the TFO purine oligonucleotide either at the 5' or on the 3' end of the oligonucleotide according to known methods, as described above for embodiment i). Preparation of the plasmid carrying the NLS peptide(s) simply requires mixing of the NLS-oligonucleotide and the homoPu-homoPy plasmid in appropriate buffer conditions, e.g. 20 mM Tris-acetate pH 7.5, 5-10 mM Mg²⁺, no K⁺ ions.

In the embodiment iii) of the invention, the NLS-oligonucleotide conjugate is inserted into the plasmid DNA by a mechanism that has been termed "strand-invading mechanism". This mechanism has been proposed to explain the binding mode of "peptide nucleic acids" (PNA) to DNA (Nielsen et al., 1991). It has been described as the strand invasion of a doublestranded DNA by an oligonucleotide (or a PNA) that leads to complementary binding between the invading molecule and the duplex through Watson-Crick interactions. Local separation of the DNA strands is required and the final conformation is thought to be a D-loop structure.

In a preferred embodiment of variant iii) of the invention, the oligonucleotide is chemically modified, thus displacement of one DNA strand is achieved by the chemically modified oligonucleotide.

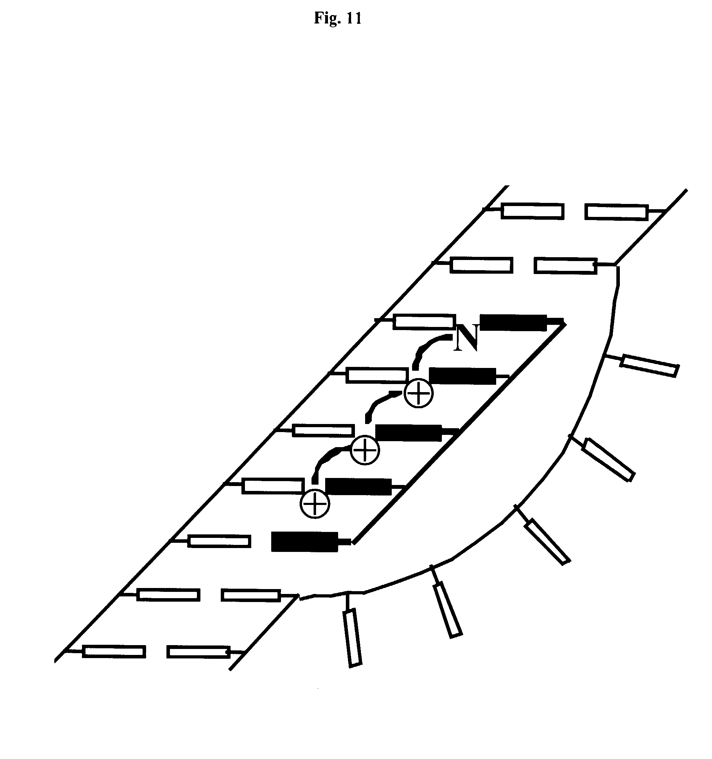

The chemical modification allows stabilization of the D-loop structure over the native duplex. As described by Schmid and Behr, 1995, invasion has been observed with synthetic oligonucleotides containing two spermine residues linked to the C2 position of inosine (Fig. 11). Stabilization of the D-loop structure is provided by spermine modifications that lie in the minor groove and clip both strands together through interstrand hydrogen bonding between each ammonium group and the nucleic bases.

Stabilization of the D-loop structure may be achieved by modifying the oligonucleotide with polycations such as spermine, spermidine or other polyamines such as (NH-(CH₂)ₙ)ₘ₋NH₂. The oligonucleotides are preferably 10-50 bases in length and contain 2-15 polyamines.

Since the strand invading mechanism is not restricted to a particular class of sequences, embodiment iii) of the invention does not require providing specific DNA sequences when constructing the plasmid. However, the oligonucleotide requires a number of bases that allow the coupling of the respective number of polyamine molecules, e.g. in the case of modifying the oligonucleotide with two spermine molecules the presence of at least two guanine bases for the chemical coupling of two spermine residues and the chemical coupling of the NLS peptide to the spermine- oligonucleotide will be useful.

The chemical modification of the oligonucleotide with the polyamine may be carried out by known methods, described by (Schmid and Behr, 1995).

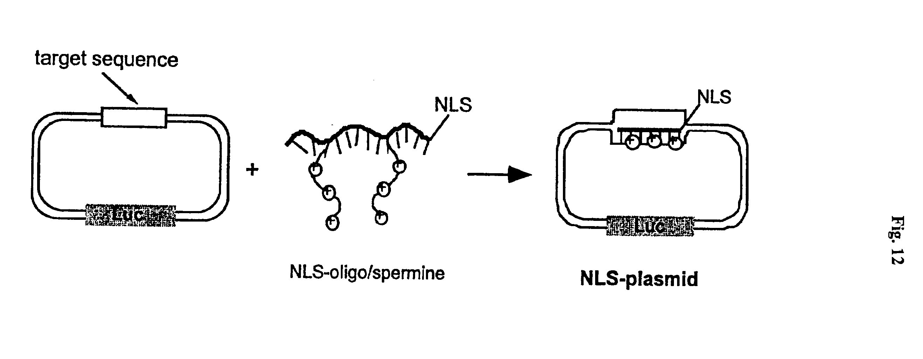

Embodiment iii) of the invention is schematically depicted in Fig. 12.

In general, the DNA molecule encodes a protein of interest to be expressed in an animal or plant cell. Alternatively, the DNA molecule may encode an inhibiting RNA molecule, e.g. an antisense RNA. In view of gene therapy applications in humans or animals, the protein of interest is a therapeutically active protein. Non limiting examples of DNA molecules are given in WO 93/07283. In addition to the coding sequence, the DNA contains regulatory sequences necessary for its expression in the cell; in the case of a plasmid, additional sequences, e.g. encoding selection markers, may be present. There are no limitations as to the sequence of the DNA. The size of the DNA is preferably in a range typical for eukaryotic genes, i.e. 1Kpb tp 1Mbp.

Transfection of the cells with the NLS-modified DNA molecules of the invention may be carried out by any standard gene delivery technology, e.g. applying the DNA molecules as such, in a suitable physiological solution, by methods known for the delivery of "naked" DNA, or by mixing the DNA containing the NLS-conjugate with a cationic lipid (Behr, 1994; Ledley, 1996), a detergent or with a cationic polymer, e.g. polyethyleneimine, which may contain a cellular targeting function and/or and endosomolytic function. Suitable methods are described in WO 93/07283, or in reviews by Ledley, 1995; Cotten and Wagner, 1993; Boussif et al.,1995.

In the case of delivery of an NLS-equipped plasmid into the cell, a cationic vector may eventually favour encounter between the plasmid and oligonucleotide sequences. In these particles, both NLS-oligonucleotides and plasmid DNA partners will be protected from nucleases.

The cells may be transfected with the NLS-DNA of the invention *in vitro*, *ex vivo* or *in vivo*. Examples for *in vivo* applications are intramuscular or intradermal injection of the NLS-DNA or electropermeabilization of NLS-DNA, both applications preferably in the absence of gene delivery vehicles, or gene gun-mediated introduction of NLS-DNA.

### Brief description of the figures:

- Fig. 1: Strategy for the preparation of a dsDNA fragment
- Fig. 2: Reaction scheme for the chemical coupling steps leading to the oligonucleotide-peptide conjugate (oligonucleotide-NLS)
- Fig.3: A. Synthesis of the oligonucleotide-NLS
B. The oligonucleotide-NLS conjugate is a substrate of proteinase K
- Fig. 4: The NLS peptide promotes high and sustained transfection levels down to 10 ng DNA
- Fig. 5: Sustained luciferase expression levels are due to the nuclear localization peptide
- Fig. 6: Reporter protein activity appears faster with CMVLuc-NLS
- Fig. 7: Hypothetical scheme of transgenic linear NLS-DNA crossing a nuclear pore
- Fig. 8: Schematic representation of triple-helix interaction
- Fig. 9: Non covalent coupling of an NLS peptide to plasmid DNA through triple helix association
- Fig. 10: Some examples of G-rich target sequences for triple-helix formation
- Fig. 11: Interaction through Watson-Crick pairing after strand-invasion of a DNA duplex by a complementary spermine-containing oligonucleotide
- Fig. 12: Non-covalent coupling of an NLS peptide to a plasmid through the strand-invading mechanism

### Example 1

### Synthesis of an oligonucleotide-NLS equipped luciferase gene; purification and proof of structure

### i) Chemicals, enzymes and oligonucleotides

4-(N-maleimidomethyl)-cyclohexane-1-carboxylic acid N-hydroxysuccinimide ester (SMCC) was purchased from Sigma. The SAL 34-mer 5' d(TCGATGTCCGCGTTGGCTT XTGCCAACGCGGACA) oligodeoxynucleotide was synthesized by Appligene using an amino-modified deoxythymidine (X; amino-modified dT, Glen Research, USA). The XMA 34-mer 5' d(CCGGCTACCTTGCGAGCTTTTGCTCGCAAGGTAG) oligodeoxynucleotide, the NLS peptide NH₂-PKKKRKVEDPYC and the mutated-NLS peptide NH₂-PK**T**KRKVEDPYC with C-terminal amidation were synthesized by Genosys using the standard F-moc chemistry for the solid phase synthesis of peptide. Hairpin structures composed of a loop of four thymines, a stem of 13 base pairs and a sticky 5'-end were formed by boiling and subsequently cooling the XMA- or the SAL- oligonucleotides in ice. *Xma*I, *Xmn*I, *Sal*I and *Bsp*HI restriction endonucleases, T4 polynucleotide kinase T4 DNA ligase and Exonuclease III were purchased from New England Biolabs (Ozyme, France). Linear 22 kDa (ExGen500) and branched 25 kDa polyethylenimines (PEI) were purchased from Euromedex (Souffelweyersheim, France) and Fluka (Saint-Quentin Fallavier, France), respectively. Transfectam® (dioctadecylamido-glycylspermine, DOGS) was synthesized as described (Behr et al., 1989).

### ii) Preparation of the oligo-peptide conjugate

2 OD units (6,46 nmoles, assuming ε²⁶⁰= 309,800 M⁻¹cm⁻¹) of the aminomodified SAL oligonucleotide in 20 µl phosphate buffer (10 mM, pH 7.5) was mixed with 40:1 molar excess of the bifunctional crosslinker SMCC in DMF (30 mM stock solution) and incubated at room temperature for 2 hours. Excess SMCC was removed using a Nick-Spin Column (Amersham-Pharmacia, Orsay, France) which had been equilibrated with PBS. The recovered oligonucleotide solution (100 µl) was immediately reacted with tenfold molar excess of NLS-peptide (or mutated-NLS-peptide) overnight at room temperature, then stored at -20°C. The oligo-peptide conjugate was purified by preparative PAGE (20% acrylamide denaturing gel containing 8 M urea. Electrophoresis was done 3 hours at 60 W). The coupling yield was 30 %, based on quantification of the radiolabeled oligonucleotides after migration in a 20% denaturing polyacrylamide gel. To assess the peptide content, 1 pmol of radiolabeled oligo-NH₂ or oligonucleotide-NLS was incubated in 10 mM Tris-HCl pH 8, 1 mM EDTA in the presence of proteinase K (0.5 mg/ml) for 10 min at 37°C, followed by incubation for 10 min at 65°C and subsequent loading on a denaturing polyacrylamide gel. Visualization and quantification were performed with a PhosphorImager 425 (Molecular Dynamics, SA).

### iii) Ligation of the CMVLuc restriction fragment to the hairpin oligonucleotides

pCMVLuc plasmid, encoding the *Photinus pyralis* luciferase under the control of the cytomegalovirus enhancer/promoter and followed by the SV40 early polyadenylation signal was prepared as described in WO 93/07283 (designated there "pCMVL"), and propagated and purified as described (Zanta et al., 1997). *Xmn*I/*Xma*I double digestion (10 U/µg DNA) was performed at 37 °C for 2 hours, followed by enzyme heat inactivation (65°C for 20 min), prior to *Sal*I cleavage (37°C for 2 hours; 10 U/µg DNA). After removal of the endonucleases by DNA precipitation, *Bsp*HI digestion was performed for 2 hours at 37°C (10 U/µg DNA). Separation of the CMVLuc-containing DNA fragment (3380 bp) from the shorter restriction fragments was performed by ultracentrifugation (30,000 rpm, 19 hours at 25°C, Beckman Ultracentrifuge L8/55, France) in a 15-30% sucrose gradient.

The 5'-end of the oligo-peptide carrying the NLS or the mutated-NLS peptide was radiolabeled with [γ-³²P]-ATP and T4 polynucleotide kinase (1 U/pmol oligonucleotide at 37°C for 30 min). The 5'-end of the oligo-cap was phosphorylated similarly with ATP. Excess [γ-³²P]-ATP and ATP were removed using Microspin G-25 columns (Amersham-Pharmacia). Prior to ligation, the hairpin form of the oligonucleotides presenting a sticky 5'-end was obtained by boiling and subsequently cooling the sample in ice. Ligation of the CMVLuc fragment with the oligo-cap and the oligo-peptide was performed overnight at 13 °C with a 15-fold molar excess of each oligonucleotide and T4 DNA ligase (10,000 U/µg DNA). The excess oligonucleotide was removed using a Microspin S-400 HR column (Amersham-Pharmacia). Quantification of the ligase reaction yield (ca. 80-90 %) was performed by Cerenkov counting (TRI-CARB 2100 TR Liquid Scintillation Analyzer, Packard, France). Capping of the CMVLuc fragment was checked by digestion with Exonuclease III (10 U/µg DNA) at 37°C. Agarose gel electrophoresis showed the uncapped and hemicapped fragments to be totally digested, whereas the capped fragment remained undigested.

If not stated otherwise, the following materials and methods were used in the Experiments described below:

### i) Cells and cell culture

NIH 3T3 murine fibroblasts were purchased from ATCC (Rockville, MA, USA) and grown in DMEM (Gibco BRL, Cergy-Pontoise, France). BNL CL.2 murine hepatocytes (ATCC) were grown in DMEM high glucose (4.5 g/l). HeLa human cervix epitheloid carcinoma cells were grown in MEM with Earle's salt (PolyLabo, Strasbourg, France). Human monocyte-derived macrophages, from an healthy donor (Hautepierre Hospital, Strasbourg) and isolated from blood by Ficoll, were grown in RPMI 1640 (Biowhittaker). Dorsal root ganglia (DRG) new born rat neurons were obtained and grown as described by Lambert et al., 1996. All cell culture media were supplemented with 10% FCS (fetal calf serum, Gibco BRL), 2 mM L-glutamine, 100 units/ml penicillin and 100 µg/ml streptomycin (Gibco BRL). Cells were maintained at 37°C in a 5% CO₂ humidified atmosphere.

### ii) Cell transfection

For each cell line used, 10,000 cells/well were seeded twenty four hours before transfection in 96 multi-well tissue culture plates (Costar, D. Dutscher, France) in order to reach 60-70% confluence during transfection. Before transfection, cells were rinsed and 0.2 ml of fresh culture medium supplemented (transfection in the presence of serum) or not (transfection in the absence of serum) with 10% FCS was added to each well. The desired amount of DNA was diluted into 46 µL (final volume) of 0.15 M NaCl or 5% glucose solutions. The desired quantity of ExGen500, 25 kDa PEI or Transfectam (from a 1 mM aqueous amine nitrogen stock solution of PEI or a 2 mM ethanolic stock solution of Transfectam) was then added to the DNA-containing solutions, vortexed gently and spun down. After 10 min, volumes corresponding to 10, 20 or 200 ng of CMVLuc fragment (10 ng/µl DNA) or to the gene-number corrected amount of plasmid DNA were added to the cells. The cell culture dish was immediately centrifuged (Sigma 3K10, Bioblock, France) for 5 min at 1500 rpm (280 g) or 500 rpm for primary neurons. After 2-3 hours, 20 µl of fetal calf serum were added to the serum-free wells. Cells were cultured for 24 hours and tested for reporter gene expression. All experiments were done in duplicate.

### iii) Luciferase Assay

Luciferase gene expression was measured by a luminescence assay. The culture medium was discarded and cell lysate harvested following incubation of cells for 30 min at room temperature in 100 µl of Lysis Reagent 1x (Promega, MA, USA). The lysate was vortexed gently and centrifuged for 5 min at 14,000 rpm at 4°C. Twenty µl of supernatant were diluted into 100 µl of luciferase reaction buffer (Promega) and the luminescence was integrated over 10 seconds (Mediators PhL, Wien, Austria). Results were expressed as light units per mg of cell protein (BCA assay, Pierce).

### Example 2

### Synthesis of a capped gene-peptide conjugate ; purification and proof of structure

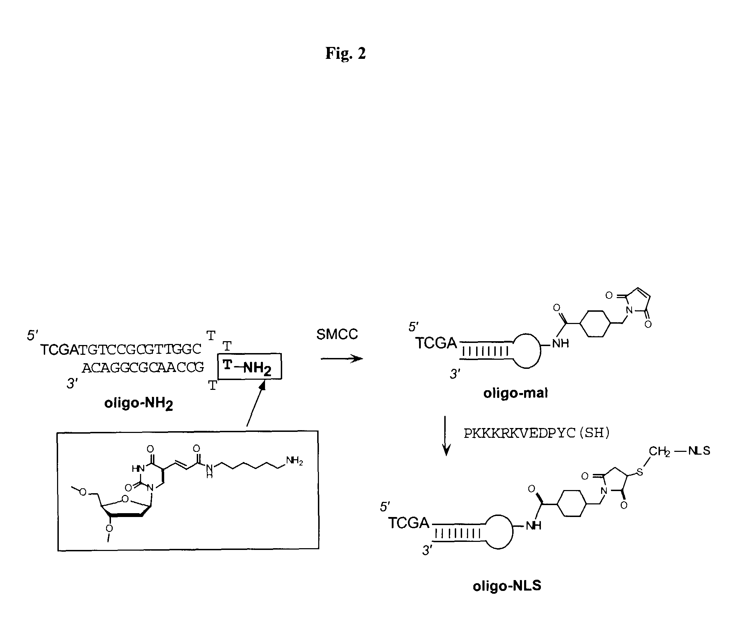

The construction was based on ligation of a pair of hairpin oligonucleotides to unique cohesive termini generated on the reporter gene, as schematically depicted in Fig. 1. Incorporation of ³²P* into the modified oligonucleotide allowed us to follow the reaction kinetics, to purify the fragment of interest and to verify its structure. The firefly luciferase reporter gene (Luc) flanked by the cytomegalovirus (CMV) enhancer/promoter sequence and the SV40 polyadenylation signal was excised from the pCMVLuc plasmid (Fig. 1). Quadruple endonuclease digestion ensured straightforward large-scale separation of the 3380 bp CMVLuc fragment from the remaining < 1 kbp fragments by ultracentrifugation through a 15-30% sucrose gradient. T₄-loops are well-suited for hairpins and the C-terminal thiol group of the PKKKRKVEDPYC peptide was conjugated to a thymine with a C(5)-amino group (Seibel et al., 1995) via an activated ester/maleimide bifunctional linker (SMCC) as detailed in Fig. 2: A hairpin oligonucleotide with a free alkylamino group in the T₄ loop (oligo-NH₂) was reacted with the heterobifunctional crosslinker SMCC to give a thiol-reactive maleimide oligonucleotide (oligo-mal) which was in turn reacted with the C-terminal cysteinamide residue of the NLS dodecapeptide).

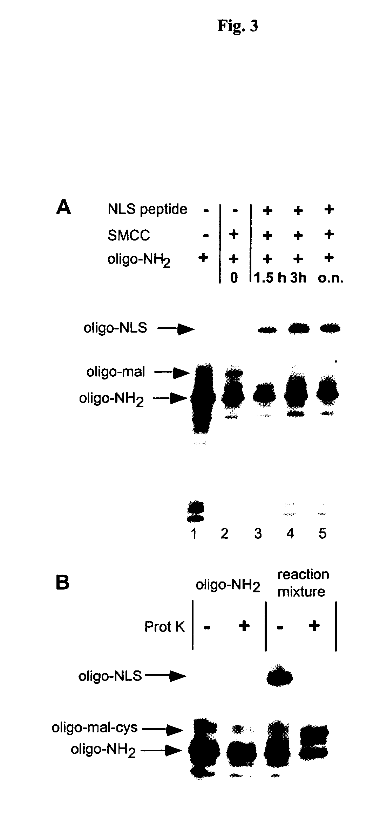

Preliminary experiments showed that the global reaction yield was highest following activation of oligo-NH₂ with SMCC for 2 h (significant parasitic maleimide hydrolysis may occur with time). Oligonucleotides 5'-³²P radiolabeling and polyacrylamide gel electrophoresis (Fig. 3A: Polyacrylamide gel electrophoresis (PAGE) analysis of the formation of the oligo-peptide conjugate. Aliquots of the reaction mixture were taken at different time intervals and analyzed on a 20% denaturing gel after radiolabeling of the oligonucleotides. Lane 1 : oligo-NH₂; lane 2: oligo-NH₂/SMCC reaction mixture after 2 h; lanes 3, 4 and 5: reaction mixture 1.5 h, 3 h or overnight after addition) showed the peptide conjugation reaction to be completed after 3 h, giving 30% oligonucleotide-NLS based on the starting oligonucleotide. Proteinase K digestion converted oligonucleotide-NLS to a faster migrating compound, presumably the oligonucleotide conjugated to the C-terminal aminoacid (oligo-mal-cys), thus establishing the chimeric nature of the conjugate (Fig. 3B: Radiolabeled oligo-NH₂ or the crude oligonucleotide-NLS reaction mixture were digested with proteinase K. Products were analyzed on a 20% denaturing gel and show total conversion of oligonucleotide-NLS into a faster migrating band, presumably oligo-mal-cys). The oligonucleotide-NLS was purified by electrophoresis. The uncapped CMVLuc fragment was then simultaneously reacted with a 15-fold excess of oligo-cap and oligonucleotide-NLS using T4 DNA ligase in previously optimized conditions. Ligation reaction yield (80-90%) was assessed by quantitative radioactivity counting and full capping of the CMVLuc fragment was checked by 3'-exonuclease digestion. CMVLuc-NLS was purified by gel permeation.

### Example 3

### Transfection with low amounts of DNA

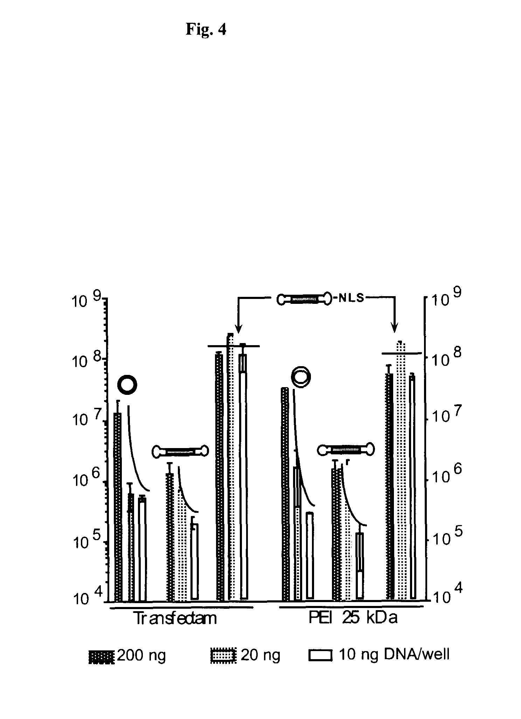

The reporter gene construct was obtained by chemical/enzymatic synthesis and purified by PAGE/gel permeation, instead of being amplified in bacteria. Only limited amounts of DNA could therefore be obtained and the transfection setup had to be miniaturized. A convenient 96-well microtiter plate assay developed by Felgner et al., 1994, was chosen. Using several optimized cationic lipid formulations, these authors showed that transfection was best at 2-0.5 µg plasmid and quickly fell off below 0.25 µg. This result was confirmed with pCMVLuc and two other cell types (Table 1, entry 4 and Fig. 4), thus putting our ultimate goal to obtaining an effective transfection with less than 200 ng DNA. As a preliminary test, the relevance to cap the reporter gene at both ends was investigated, thus avoiding free DNA ends that are prone to exonuclease-mediated DNA degradation and to eliciting a DNA repair response. To this end, the uncapped, hemicapped and fully capped CMVLuc DNAs (200 ng) were transfected into hepatocytes. (BNL CL.2 hepatocytes were transfected with 200 ng DNA/well. Complexes were formed with 25 kDa PEI (N/P= 10) in 150mM NaCl, and in the absence of serum.)
Luciferase activities (Table 1) showed both un- and hemi-capped molecules to give 25-fold less gene expression than the capped one thus justifying the choice of our chemical strategy. Thus,this is experiment shows that free DNA ends decrease transfection efficacy. (*a*: Transfection with 20 ng plasmid.)

### Example 4

The NLS peptide allows effective transfection with minute quantities of DNA

3T3 cells were transfected with decreasing amounts of DNA, using the NLS-bearing capped gene (CMVLuc-NLS), the capped gene (CMVLuc) and the corresponding mass-corrected amount of plasmid DNA (pCMVLuc). While efficacy very much decreased for `20 ng' plasmid DNA (in fact 38 ng), as expected (see above), transfection levels remained remarkably high and constant over the 3T3 cells were transfected with decreasing amounts of DNA complexed in 150 mM NaCl to a cationic lipid (Transfectam, N/P=6) or to a cationic polymer (25 kDa PEI N/P=10) in the absence of serum.range 200-10 ng DNA with CMVLuc-NLS (Fig. 4: 3T3 cells were transfected with decreasing amounts of DNA complexed in 150 mM NaCl to a cationic lipid (Transfectam, N/P=6) or to a cationic polymer (25 kDa PEI N/P=10) in the absence of serum.). Comparison with the capped gene lacking the NLS peptide showed 100-1000 fold more expression when the nuclear localization signal peptide was present on the gene. Similar conclusions were obtained, whether using a cationic lipid (Transfectam, Fig. 4 left) or a cationic polymer (branched PEI, Fig. 4 right). Further data (not shown) confirmed that other cationic carrier molecules behaved similarly, but also that efficacy dropped fiftyfold within the range 10-1 ng CMVLuc-NLS.

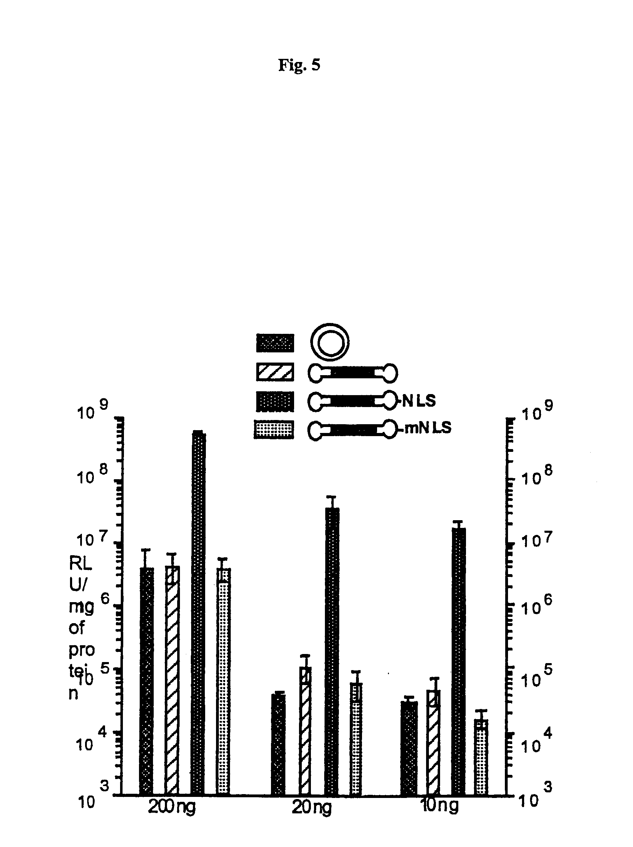

Improved transfection involves the cellular nuclear import machinery. Comparative experiments between CMVLuc and CMVLuc-NLS, although interesting, do not really allow one put forward any hypothesis concerning the mechanism. Nuclear import of DNA and oligonucleotides has mainly been followed with fluorescent tags and FISH, in microinjected or digitonin-permeabilized cells. Proof of active import relied on inhibition experiments using energy-decoupling molecules, nuclear pore-binding agglutinins or mutations in the signal peptide sequence. Gene expression is an unambiguous proof of nuclear localization and transfection can be regarded as a straightforward way of introducing DNA into intact cells. The Lysine ⇒ Threonine mutation abolishes nuclear import (Kalderon et al., 1984). Therefore, a capped linear DNA molecule was synthesized (CMVLuc-mNLS) with a mutated PKTKRKVEDPYC sequence. Comparative transfection experiments with HeLa cells are shown in Fig. 5 (HeLa cells were transfected with various amounts of DNA complexed to ExGen500 PEI (N/P=5 in a 5% glucose solution) in the presence of 10% serum.). Irrespective of the amount of DNA used, the Lys ⇒ Thr mutation brought transfection down to the level obtained with the capped gene having no peptide at all, confirming the functionality of the NLS/importin-α interaction.

### Example 5

### NLS peptide-mediated transfection enhancement is a general phenomenon

A series of comparative experiments was undertaken with various cell types. Transfection enhancement due to the presence of the NLS peptide was always observed (Table 2, which shows the average enhancement of transfection with CMVLuc-NLS.Values refer to transfection with 10-20 ng DNA/well, in the absence of serum. *a*. Estimated spread ± 30%; b. In the presence of 10% serum during transfection; c. In 24-well plates using 200 ng DNA in the presence of 10% serum.)

However enhancement factors were spread widely with cell type (10-1000-fold), with no obvious relation with tissue origin nor cell type (primary *vs*. transformed cells). Nondividing primary cells such as human monocyte-derived macrophages and rat dorsal root ganglia neurons showed less impressive enhancement than 3T3 and HeLa cells. However, similar values were also seen for the easily transfected and fast dividing rat hepatocyte-derived cell line BNL CL.2.
The general experimental setup for transfection included 96-well microtiter plates and no serum during 2 hours following addition of the DNA/vector complexes to the cells. Several experiments were also performed on a larger scale (24-well plates) or in the presence of 10% serum during transfection. Table 2 shows the average enhancement of transfection with CMVLuc-NLS.
(Values refer to transfection with 10-20 ng DNA/well, in the absence of serum. *a*: estimated spread ± 30%; b: in the presence of 10% serum during transfection; c: in 24-well plates using 200 ng DNA in the presence of 10% serum.). The results obtained (Table 2, b and c) confirmed the general conclusions derived with the 96-well setup.

### Example 6

### Time course of the transgene expression

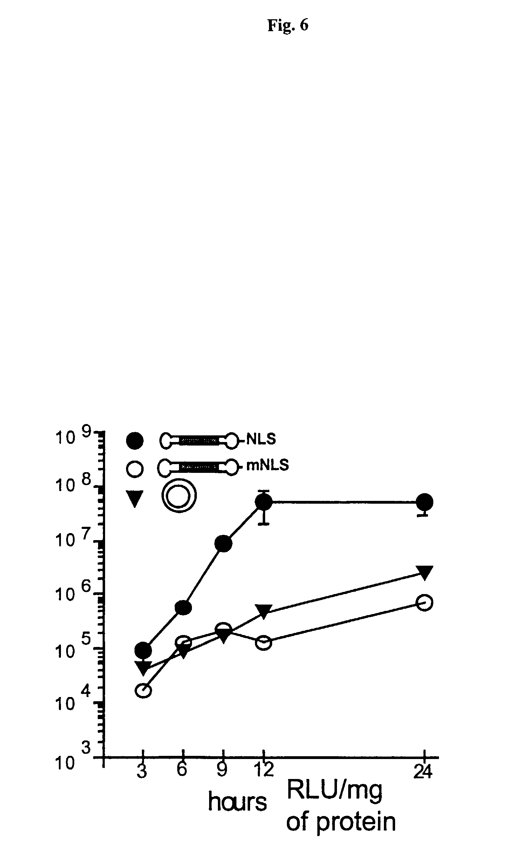

Active transport of the reporter gene via the nuclear import machinery could result in faster appearing expression. More interesting, a lower intracellular barrier to gene delivery means less DNA/vector complexes required in the cytoplasm, hence lower toxicity and more sustained expression. The kinetics of gene expression was followed in detail up to 24 h (Fig. 6: HeLa cells were transfected with 10 ng DNA complexed to ExGen 500 PEI (N/P=5 in 5% glucose solution) in the absence of serum. Cells were lysed and luciferase activity was measured at the indicated time after transfection. The absence of error bars indicates errors that are smaller than the label on the graph.) and then daily up to 3 days. Again, transfection with CMVLuc-NLS was much more effective than with CMVLuc-mNLS. Remarkably (and reproducibly), NLS-driven transfection reached its plateau after 12 h, whereas transfection levels obtained with the plasmid or the mutated NLS sequence were still increasing significantly up to 24 h. Previous experiments (Pollard et al., 1998) showed both transfection and cytoplasmic injection of DNA/PEI complexes to have superimposable kinetics of transgene expression, suggesting that the slow step was intracellular trafficking/nuclear entry rather than cell entry. Using the nuclear import machinery seems to speed up the rate-limiting step. For longer time periods, expression stayed broadly constant with a low standard deviation. However, the rather large variability observed upon repeating this experiment prevented any conclusion from being drawn (the inconsistent results observed at t >1d may be due to the fact that cells reached confluency at day 1).

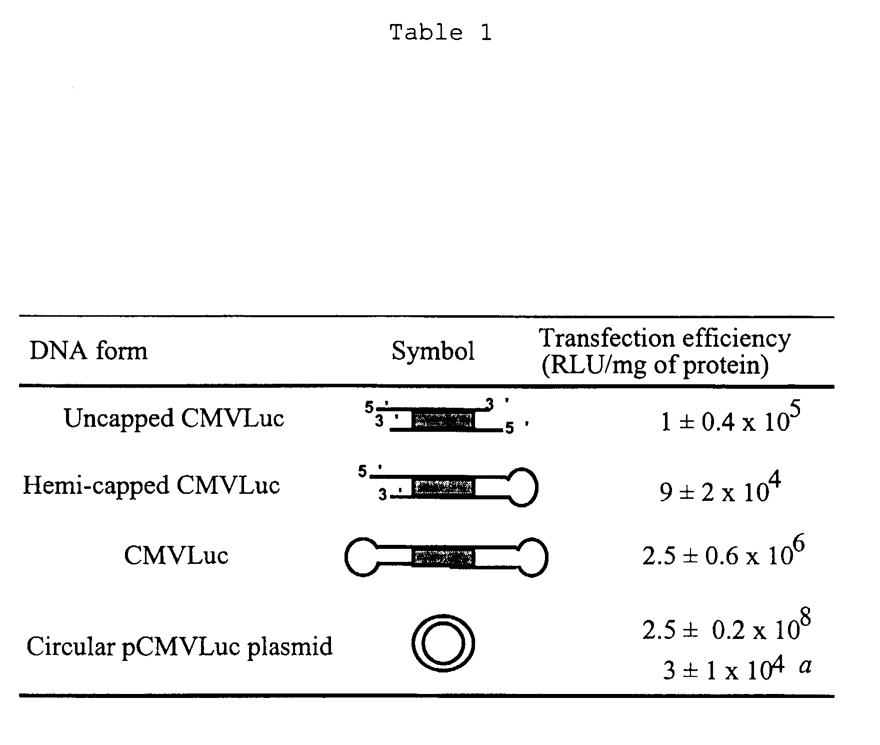

**Table 2**

| **CELL TYPE** | **Enhancement factor** ^{***a***} |
|---|---|
| BNL CL.2 | 10 |
| 3T3 | 400^{***b***}- 1000 |
| HeLa | 200^{***c***}- 300 |
| Murine DRG neurons | 30 |
| Human macrophages | 10 |

### REFERENCES

Adam, S. A. & Gerace, L. (1991) *Cell* **66**, 837-847.
Behr, J. P., Demeneix, B., Loeffler, J. P. & Perez-Mutul, J. (1989) *Proc. Natl. Acad. Sci. USA* **86**, 6982-6986.
Behr, J. P., (1994), *Bioconjugate Chemistry,* 5, 382-389
Blessing, T., Remy, J. S. & Behr, J. P. (1998) *Proc. Natl. Acad. Sci. USA* **95**, 1427-1431.
Boussif, O., Lezoualch, F., Zanta, M. A., Mergny, M. D., Scherman, D., Demeneix, B. & Behr, J. P. (1995) *Proc. Natl. Acad. Sci. USA* **92,** 7297-7301.
Cereghini, S. & Yaniv, M. (1984) *Embo J.* **3**, 1243-1253.
Citovsky, V., et al., (1992), *Science* 256: 1802
Collas, P., Husebye, H. & Aleström, P. (1996) *Transgenic Res.* **5**, 541-548.
Cotten, M. and Wagner, E., (1993), Curr. Opin. Biotech., 4, 705-710
Dean, D. A. (1997) *Exp. Cell Res.* **230**, 293-302.
Debin, A., Malvy, C., and Svinarchuk, F. (1997) *Nucleic Acids Res.* **25**, 1965-1974.
Dowty, M. E., Williams, P., Zhang, G., Hagstrom, J. E. & Wolff, J. A. (1995) *Proc. Natl. Acad. Sci. USA* **92,** 4572-4576.
Felgner, J. H., Kumar, R., Sridhar, C. N., Wheeler, C. J., Tsai, Y. J., Border, R., Ramsey, P., Martin, M. & Felgner, P. L. (1994) J. *Biol. Chem.* **269,** 2550-2561.
Fominaya, J. & Wels, W. (1996) J. *Biol. Chem.* **271,** 10560-10568.
Fritz, J. D., Herweijer, H., Zhang, G. F. & Wolff, J. A. (1996) *Hum. Gene Ther.* **7,** 1395-1404.
Garcia-Bustos, et al., (1991) *Biochem. Biophys. Acta* 1071: 83
Gorlich, D. & Mattaj, I. W. (1996) *Science* **271,** 1513-1518.
Greber, U. F., Suomalainen, M., Stidwill, R. P., Boucke, K., Ebersold, M. W. & Helenius, A. (1997) *EMBO J.* **16,** 5998-6007.
Greber, U. F. (1998) in *Self-assembling complexes for gene delivery : from laboratory to clinical trial,* eds. Kabanov, A. V., Felgner, P. L. & Seymour, L. W. (John Wiley & Sons Ltd, Chichester), pp. 89-114.
Hagstrom, J. E., Ludtke, J. J., Bassik, M. C., Sebestyén, M. G., Adam, S. A. & Wolff, J. A. (1997) *J. Cell Sci.* **110,** 2323-2331.
Jeong, S. & Stein, A. (1994) *Nucleic Acids Res.* **22,** 370-375.
Kalderon, D., Roberts, B. L., Richardson, W. D. & Smith, A. E. (1984) *Cell* **39,** 499-509.
Kaneda, Y., Iwai, K. & Uchida, T. (1989) *Science* **243,** 375-378.
Labat-Moleur, F., Steffan, A. M., Brisson, C., Perron, H., Feugeas, O., Furstenberger, P., Oberling, F., Brambilla, E. & Behr, J. P. (1996) *Gene Therapy* **3,** 1010-1017.
Lambert, R. C., Maulet, Y., Dupont, J. L., Mykita, S., Craig, P., Volsen, S. & Feltz, A. (1996) *Mol Cell Neurosci* **7,** 239-246.
Langle-Rouault, F., Patzel, V., Benavente, A., Taillez, M., Silvestre, N., Bompard, A., Sczakiel, G., Jacobs, E. & Rittner, K. (1998) *J. Virol.* **72,** 6181-6185.
Le Doan, T., Perrouault, L., Praseuth, D., Habhoub, N., Decout, J., Thuong, N. T., Lhomme, J. & Hélène, C. (1987) *Nucleic Acids Res.* **15,** 7749.
Ledley, F. D., (1995), Human Gene Ther., 6, 1129-1144
Ledley, F. D., (1996), Pharmaceut Res, 13, 1595-1614.
Luby-Phelps, K. (1994) *Curr. Opin. Cell Biol.* **6,** 3-9.
Maul, G. G. & Deaven, L. (1977) *J. Cell Biol.* **73,** 748-760.
Melchior, F. & Gerace, L. (1998) *Trends Cell. Biol.* **8,** 175-179.
Mirzayans, R., Aubin, R. A. & Paterson, M. C. (1992) *Mutat. Res.* **281,** 115-122.
Mislick, K. A. & Baldeschwieler, J. D. (1996) *Proc. Natl. Acad. Sci. USA* **93,** 12349-12354.
Moser, H. E. & Dervan, P. B. (1987) *Science* **238,** 645.
Nakanishi, M., Weber, K. T., and Guntaka, R. V. (1998) *Nucleic Acids Res.* **26,** 5218-5222.
Nielsen, P. E., Egholm, M., Berg, R. H. & Buchardt, O. (1991) *Science* **254,** 1497-1500.
Nigg, E. A. (1997) *Nature* **386,** 779-787.
Ohno, M., Fornerod, M. & Mattaj, I. W. (1998) *Cell* **92,** 327-336.
Panté, N. & Aebi, U. (1995) *J. Cell Sci. Suppl.* **19,** 1-11.
Plank, C., Oberhauser, B., Mechtler, K., Koch, C. & Wagner, E. (1994) *J. Biol. Chem.* **269,** 12918-12924.
Pollard, H., Remy, J. S., Loussouarn, G., Demolombe, S., Behr, J. P. & Escande, D. (1998) *J. Biol. Chem.* **273,** 7507-7511.
Rando R. F., DePaolis L., Durland R.H., Jayaraman K., Kessler D.J., Hogan M.E., (1994), *Nucleic Acids Res.* Feb 25;22(4):678-85.
Remy, J. S., Kichler, A., Mordvinov, V., Schuber, F. & Behr, J. P. (1995) *Proc. Natl. Acad. Sci. USA* **92,** 1744-1748.
Sambrook, J., Fritsch, E.F. and Maniatis, T., 1989, Molecular Cloning, Cold Spring Harbor Laboratory Press (2. Edition).
Sandler, et al., (1989) *J. Cell Biol.* 109: 2665
Schmid, N. & Behr, J. P. (1995) *Tetrahedron Lett.* **36,** 1447-1450
Sebestyén, M. G., Ludtke, J. J., Bassik, M. C., Zhang, G., Budker, V., Lukhtanov, E. A., Hagstrom, J. E. & Wolff, J. A. (1998) *Nature Biotech.* **16,** 80-85.
Seibel, P., Trappe, J., Villani, G., Klopstock, T., Papa, S. & Reichmann, H. (1995) *Nucleic Acids Res.* 23, 10-17.
Svinarchuk, F., Bertrand, J. R., and Malvy, C. (1994) *Nucleic Acids Res.* **22,** 3742-3747.
Xu, Y. H. & Szoka, F. C. (1996) *Biochemistry* **35,** 5616-5623.
Zabner, J., Fasbender, A. J., Moninger, T., Poellinger, K. A. & Welsh, M. J. (1995) *J. Biol. Chem.* **270,** 18997-19007.
Zanta, M. A., Boussif, O., Adib, A. & Behr, J. P. (1997) *Bioconjugate Chem.* **8,** 839-844.

## Claims

1. A non-viral transfection vector comprising a DNA molecule which is to be delivered into the nucleus of a cell, characterized that said DNA molecule is equipped with 1 to 15 conjugates comprising a nuclear localization signal (NLS) covalently linked to an oligonucleotide, each of which NLS conjugate has been
i) covalently linked to one or both termini of a linear DNA molecule, or
ii) associated with a plasmid DNA molecule by forming a triple helix, or
iii) inserted in a plasmid DNA molecule by strand invasion.

2. The transfection of claim 1, wherein said DNA molecule is equipped with 1 to 5 NLS conjugates.

3. The transfection vector of claim 1, characterized in that the linear DNA molecule defined in i) is equipped, at both ends, with an oligonucleotide hairpin, one or both hairpins being conjugated to an NLS peptide.

4. The transfection vector of claim 3 which contains a single NLS peptide.

5. The transfection vector of claim 1, wherein the plasmid DNA molecule defined in ii) is associated with an NLS conjugated to an oligonucleotide which recognizes a homopurine or a homopyrimidine sequence within the plasmid in a sequence specific manner.

6. The transfection vector of claim 5, wherein the oligonucleotide is a homopurine.

7. The transfection vector of claim 1, wherein the plasmid DNA defined in iii) is equipped with an NLS conjugated to an oligonucleotide that is chemically modified such that the the D-loop formed through strand invasion is stabilized.

8. The transfection vector of claim 7, wherein the oligonucleotide is modified with spermine.

9. A pharmaceutical composition containing, as an active ingredient, a transfection vector of claim 1, wherein said DNA molecule encodes a therapeutically active protein.

10. A method of transfecting a cell, wherein a transfection vector defined in claim 1 is complexed with a cationic compound and the cells are contacted with the complex.


### Annex to the description

SEQUENCE LISTING

(1) GENERAL INFORMATION:

(i) APPLICANT:
(A) NAME: Boehringer Ingelheim International GmbH
(B) STREET: Binger Strasse 173
(C) CITY: Ingelheim am Rhein
(E) COUNTRY: Germany
(F) POSTAL CODE (ZIP): 55216
(G) TELEPHONE: 06132/772282
(H) TELEFAX: 06132/774377

(ii) TITLE OF INVENTION: Non-viral transfection vector

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(2) INFORMATION FOR SEQ ID NO:1:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: synthetic desoxyribonucleotide

(xi) SEQUENCE DESCRIPTION: SEQ ID NO:1:

TCGATGTCCG CGTTGGCTTX TGCCAACGCG GACA 34

(2) INFORMATION FOR SEQ ID NO:2:

(i) SEQUENCE CHARACTERISTICS:
(A) LENGTH: 34 base pairs
(B) TYPE: nucleic acid
(C) STRANDEDNESS: single
(D) TOPOLOGY: linear

(ii) MOLECULE TYPE: synthetic desoxyribonucleotide

(xi) SEQUENCE DESCRIPTION: SEQ ID NO:2:

CCGGCTACCT TGCGAGCTTT TGCTCGCAAG GTAG 34

(2) INFORMATION FOR SEQ ID NO:3:

(i) SEQUENCE CHARACTERISTICS:
(A) LENGTH: 12 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:3:

Pro Lys Lys Lys Arg Lys Val Glu Asp Pro Tyr Cys
1               5                   10

(2) INFORMATION FOR SEQ ID NO:4:

(i) SEQUENCE CHARACTERISTICS:
(A) LENGTH: 12 amino acids
(B) TYPE: amino acid
(C) STRANDEDNESS: single
(D) TOPOLOGY: linear

(ii) MOLECULE TYPE: peptide

(xi) SEQUENCE DESCRIPTION: SEQ ID NO:4:

Pro Lys Thr Lys Arg Lys Val Glu Asp Pro Tyr Cys
1               5                   10